# EUROPÄISCHE PATENTANMELDUNG

(11) **EP 3 545 940 A2**
(43) Veröffentlichungstag der Anmeldung: **02.10.2019**
(21) Anmeldenummer: 19405004.3
(22) Anmeldetag: 28.01.2019
(51) Int. Cl.: A61K 6/09, C07C 271/10, C08G 18/81

(54) **DENTALMATERIALIEN MIT ERHÖHTER DEGRADATIONSSTABILITÄT**

(30) Priorität: 08.03.2018 CH 2832018
(71) Anmelder: Saremco Dental AG, 9445 Rebstein (CH)
(72) Erfinder: Schmid, Franca, 9000 St. Gallen (CH); Evers, Christoph, 9445 Rebstein (CH); Marti, Roger, 1717 St. Ursen (CH); Vanoli, Ennio, 1720 Corminboeuf (CH); Piantini, Umberto, 1971 Grimisuat (CH); Schalcher, Christophe, 1700 Fribourg (CH)

(57) **Zusammenfassung**

Diese Erfindung betrifft dentale Restaurationsmaterialien auf Methacrylatbasis, welche sich durch eine verbesserte Degradationsstabilität gegenüber dem Stand der Technik auszeichnen. Die Aufgabe wurde durch den Einsatz eines neuen, speziellen, polymerisierbaren Monomers gelöst. Überraschend wurde gefunden, dass der Einsatz von einem Reaktionsprodukt aus einem mit einem aromatischen Kern und mindestens zwei Hydroxy-Gruppen bestehenden Dimethacrylat mit 2 unterschiedlichen Isocyanaten sehr degradationsbeständige dentale Restaurationsmaterialien liefert.

## Beschreibung

### Hintergrund der Erfindung

Diese Erfindung betrifft dentale Restaurationsmaterialien auf Methacrylatbasis, welche sich durch eine verbesserte Degradationsstabilität gegenüber dem Stand der Technik auszeichnen.

Degradationsprozesse bei dentalen Restaurationsmaterialien auf Methacrylatbasis sind seit Jahren bekannt. Seit den frühen 1970-Jahren haben viele Forschungsgruppen die Degradation von dentalen Füllungskunststoffen untersucht. Bei diesen Untersuchungen wurde in erster Linie auf den Materialverlust in Folge von Abrasions- und mechanischer Belastung fokussiert (Lee HL, Swartz ML, Smith FF.; Physical properties of four thermosetting dental restorative resins. J Dent Res 1969;48(4):526-35.; Truong VT, Tyas MJ. Prediction of in vivo wear in posterior composite resins: a fracture mechanics approach. Dent Mater 1988;4(6):318-27; Taylor DF, Bayne SC, Sturdevant JR, Wilder AD. Comparison of direct and indirect methods for analyzing wear of posterior composite restorations. Dent Mater 1989;5(3):157-60.) Bei den dentalen Adhäsiven sind hydrolytische Degradationsprozesse bekannt, die aber in erster Linie in einem sehr sauren Milieu stattfinden, vor allen bei hydrophilen Monomeren gefunden werden und sich negativ auf die Lagerstabilität dieser Adhäsive auswirken. (Van Landuyt, Kirsten L., et al. "Systematic review of the chemical composition of contemporary dental adhesives." Biomaterials 28.26 (2007): 3757-3785).

Anfang der 1990er Jahre verlagerte sich ein Schwerpunkt der Studien jedoch auf den chemischen Abbau dieser Füllungsmaterialien, da vermutet wurde, dass Enzyme in der Mundhöhle zum Abbau von dentalen Restaurationsmaterialien auf Methacrylatbasis beitragen könnten (Winkler M, Greener EH, Lautenschlager EP. Non-linear in vitro wear of posterior composites with time. Dent Mater 1991;7(4):258-62; Freund M, Munksgaard EC. Enzymatic degradation of BisGMA/ TEGDMA-polymers causing decreased microhardness and greater wear in vitro. Scand J Dent Seither wurde in mehreren Studien der Abbau von Harzkompositen in Gegenwart von Speichelähnlichen Enzymen (Larsen IB, Freund M, Munksgaard EC. Change in surface hardness of BisGMA/TEGDMA polymer due to enzymatic action. J Dent Res 1992;71(11):1851-3; Finer Y, Jaffer F, Santerre JP. Mutual influence of cholesterol esterase and pseudocholinesterase on the biodegradation of dental composites. Biomaterials 2004;25(10): 1787-93; Finer Y, Santerre JP. Biodegradation of a dental composite by esterases: dependence on enzyme concentration and specificity. J Biomater Sci Polym Ed 2003;14(8):837-49; Finer Y, Santerre JP. The influence of resin chemistry on a dental composite's biodegradation. J Biomed Mater Res A 2004;69(2):233-46) und die anschließende biologische Wirkung der Nebenprodukte auf die umgebenden Bakterien- und Säugetierzellen untersucht (Khalichi P, Singh J, Cvitkovitch DG, Santerre JP., The influence of triethylene glycol derived from dental composite resins on the regulation of Streptococcus mutans gene expression. Biomaterials 2009;30(4):452-9; Reichl FX, Esters M, Simon S, Seiss M, Kehe K, Kleinsasser, N, et al. Cell death effects of resin-based dental material compounds and mercurial in human gingival fibroblasts.Arch Toxicol 2006;80(6):370-7; Moharamzadeh K, Van Noort R, Brook IM, Scutt AM., Cytotoxicity of resin monomers on human gingival fibroblasts and HaCaT keratinocytes. Dent Mater 2007;23(1):40-4.; Chang MC, Lin LD, Chan CP, Chang HH, Chen LI, Lin HJ, et al. The effect of BisGMA on cyclooxygenase-2 expression, PGE2 production and cytotoxicity via reactive oxygen species- and MEK/ERKdependent and independent pathways. Biomaterials 2009;30(25):4070-7; Aranha AM, Giro EM, Hebling J, Lessa FC, Costa CA. Effects of light-curing time on the cytotoxicity of a restorative composite resin on odontoblast-like cells. J Appl Oral Sci 2010;18(5):461-6; Chang MC, Lin LD, Chuang FH, Chan CP, Wang TM, Lee JJ, et al., Carboxylesterase expression in human dental pulp cells: role in regulation of BisGMA-induced prostanoid production and cytotoxicity. Acta Biomater 2012;8(3):1380-7; Martins CA, Leyhausen G, Geurtsen W, Volk J. Intracellular glutathione: a main factor in TEGDMA-induced cytotoxicity? Dent Mater 2012;28(4):442-8).

Die Literatur zeigt, dass der enzymatische Abbau in kommerziellen, dentalen Restaurationsmaterialien auf Methacrylatbasis trotz verschiedener Methacrylat-Zusammensetzungen immer auftritt. Die Produktion dieser Abbauprodukt, die Tiefe und das räumliche Volumen der damit möglichen bakteriellen Zellpenetration innerhalb der Grenzfläche Kunststofffüllung zur Zahnhartsubstanz legen nahe, dass der biologische Abbau von dentalen Restaurationsmaterialien auf Methacrylatbasis zur Bildung von Karies-Rezidiven beitragen kann (Kermanshahi, S., Santerre, J. P., Cvitkovitch, D. G., & Finer, Y. (2010). Biodegradation of resin-dentin interfaces increases bacterial microleakage. Journal of dental research, 89(9), 996-1001). Diese biologischen Prozesse, die kommerzielle dentale Restaurationsmaterialien auf Methacrylatbasis und deren Adhäsive anfällig für vorzeitiges Versagen machen, sind derzeit außerhalb der Kontrolle des Behandlers/Zahnarztes. Daher ist es eine Aufgabe dieser Erfindung dentale Restaurationsmaterialien auf Methacrylatbasis zur Verfügung zu stellen, welches über eine verbesserte Degradationsstabilität gegenüber dem Stand der Technik verfügt.

### Stand der Technik

Eine Patentrecherche über die enzymatische Degradation von dentale Restaurationsmaterialien auf Methacrylatbasis ergab keinen Treffer. Allerdings sind aus der Wissenschaft einige Untersuchungen bekannt. Frühere Studien haben gezeigt, dass Esterasen, insbesondere Cholesterinesterase (CE), ein aus entzündlichen Zellen gewonnenes Enzym, und andere identifizierte Speichelesterasen, die Matrixkomponenten aus kommerziellen und Modell-Restaurationsmaterialien auf Methacrylatbasis hydrolysieren können (J. P. Santerre, L. Shajii and H. Tsang, Biodegradation of Commercial Dental Composites by Cholesterol Esterase, J. Dent. Res. 78, 1459 (1999), L. Shajii and J. P. Santerre, Effect of filler content on the profile of released biodegradation products in micro-filled bis-GMA/TEGDMA dental composite resins Biomaterials 20, 1897 (1999), 6. D. M. Yourtee, R. E. Smith, K. A. Russo, S. Burmaster, J. M. Cannon, J. D. Eick and E. L. Kostoryz, The stability of methacrylate biomaterials when enzyme challenged: kinetic and systematic evaluations, J. Biomed. Mater. Res. 57, 522 (2001).

Bei der Untersuchung von kommerziellen dentalen Restaurationsmaterialien auf Methacrylatbasis zeigten diejenigen Produkte, die ein Urethan-modifiziertes BisGMA (Bisphenylglycidyldimethacrylat) / TEGDMA (Triethylenglykoldimethacrylat) (ubis) -Monomersystem enthielten, eine 10-fache Verringerung der Freisetzung der Abbauprodukte (ein BisGMA-Derivat: Bishydroxypropoxyphenylpropan [bisHPPP]), verglichen mit dem auf BisGMA / TEGDMA (bis) basierten Restaurationsmaterialien auf Methacrylatbasis nach Inkubation mit Cholesterinesterase (CE). In den Sicherheitsblättern ist für das Urethan-modifiziertes BisGMA die CAS-Nr. 126646-17-1 (Urethane modified Bis-GMA dimethacrylate resin) zu finden.

Unglücklicherweise unterschieden sich diese Materialien auch hinsichtlich des Füllstofftyps und -inhalts wesentlich voneinander, was es unmöglich machte, irgendeinen spezifischen Parameter direkt mit den Unterschieden im biologischen Abbaugrad in Beziehung zu setzen. Durch die Kontrolle des Füllstoffgehalts und -typs (Finer Y, Santerre JP The influence of resin chemistry on a dental composite's biodegradation. J Biomed Mater Res A 2004: 69:233-246).wurden die biomolekularen Wechselwirkungen zwischen der Harzzusammensetzung und der Esterase-Aktivität untersucht, um die beobachteten Unterschiede in den biologischen Abbaupegeln zwischen den ubis- und den Bis-Harzsystemen zu erklären. Auch hier zeigten die Ubis-Systeme eine deutlich bessere Degradationsstabilität gegenüber den Bis-Systemen. Da beide Systeme mit Ausnahme ihrer Monomersysteme identisch waren, wurde geschlossen, dass Änderungen der Biostabilität mit der Chemie der Monomere verbunden ist.

Zu dem urethanmodifizierten BisGMA lassen sich mehrere Patente finden (GB000001465897, US000003629187, US000003629187, US000004553940, US000005876210, DE 2126419....). In keinem dieser Patente wird in irgendeiner Form über die Degradationsstabilität dieser Monomere berichtet.

Daher ist es eine Aufgabe dieser Erfindung dentale Restaurationsmaterialien auf Methacrylatbasis zur Verfügung zu stellen, welche über eine verbesserte Degradationsstabilität gegenüber dem Stand der Technik verfügen. Es dürfte unstreitig sein, dass ein Material mit erhöhter Degradationsstabilität in vielerlei Hinsicht Vorteile gegenüber den jetzt auf dem Markt verfügbaren, dentalen Restaurationsmaterialien auf Methacrylatbasis hat. Die Degradationsprodukte, die zum großen Teil noch unerforscht sind, gelangen bis her mehr oder weniger unkontrolliert in den Körper des Patienten, wobei die weitere Verstoffwechselung noch nicht gekärt ist. Zudem sollte so ein Material speziell an der Grenzfläche Kunststofffüllung zur Zahnhartsubstanz durch die bessere Degradationsstabilität ein mögliches Eindringen von Bakterien (in Folge von Degardationsprozessen) besser verhindern können und somit Sekundärkaries vorbeugen.

Die Aufgabe wurde durch den Einsatz eines neuen, speziellen, polymerisierbaren Monomers gelöst, welches in den Tests deutlich bessere Werte für die Degradation verglichen mit kommerziellen Produkten lieferte. Überraschend wurde gefunden, dass der Einsatz von einem Reaktionsprodukt aus einem mit einem aromatischen Kern und mindestens zwei Hydroxy-Gruppen bestehenden Dimethacrylat mit 2 unterschiedlichen Isocyanaten sehr degradationsbeständige dentale Restaurationsmaterialien liefert.

Beispiele für das Dimethacrylat mit einem aromatischen Kern und mindestens zwei Hydroxy-Gruppen sind HYDROCHINON DIGLYCEROLATE DIMETHACRYLATE, RESORCINOL DIGLYCEROLATE DIMETHACRYLATE, BISPHENOL A GLYCEROLATE DIMETHACRYLATE, BISPHENOL B GLYCEROLATE DIMETHACRYLATE, BISPHENOL C GLYCEROLATE DIMETHACRYLATE, BISPHENOL E GLYCEROLATE DIMETHACRYLATE, BISPHENOL F GLYCEROLATE DIMETHACRYLATE, wobei diese Aufzählung keinen Anspruch auf Vollzähligkeit erhebt. Besonders bevorzugt ist das BISPHENOLA GLYCEROLATE DIMETHACRYLATE (BisGMA).

In einem ersten Reaktionsschritt wird dieses Methacrylat mit einem Diisocyanat umgesetzt. Beispiele für solche Diisocyanate sind z. B.: Alkylendiisocyanate, Äthylendiisocyanat, Propylendiisocyanat, Tetramethylendiisocyanat, Pentamethylendiisocyanat, Hexamethylendiisocyanat (HDI), Trimethylhexamethylene diisocyanat (TMDI), Octamethylendiisocyanat, Decamethylenediisocyanat, Undecamethylendiisocyanat, Dodecamethylendiisocyanat, oder Arylene-Diisocyanate wie Xylylene-1,4-Diisocyanat, Xylylene-1,5-Diisocyanat, m-Phenylenediisocyanat, p-Phenylenediisocyanat, Toluol-2,4-Diisocyanat, Toluol-2,6-Diisocyanat, Mesitylenediisocyanat, Durylenediisocyanat, 1-Methylphenylene-2,4-Diisocyanat, Naphthylene-1,4-Diisocsanat, Naphthylene-1,5-Diisocyanat. Auch diese Aufzählung erhebt keinen Anspruch auf Vollständigkeit. Besonders bevorzugt sind Toluol-2,4-Diisocyanat, Toluol-2,6-Diisocyanat, Trimethylhexamethylene diisocyanat (TMDI) und Hexamethylendiisocyanat (HDI).

Dabei sind Einwaagenverhältnisse von Dimethacrylat mit einem aromatischen Kern und mindestens zwei Hydroxy-Gruppen zu Diisocyanat von 1 mol: 0.01 mol bis 1 mol: 0.9 mol möglich. Bevorzugt sind Einwaagenverhältnisse von Dimethacrylat mit einem aromatischen Kern und mindestens zwei Hydroxy-Gruppen zu Diisocyanat von 1 mol : 0.2 mol bis 1 mol:0.5 mol, besonders bevorzugt 1 mol: 0.3 mol bis 1 mol :0.4 mol.

In einem zweiten Schritt erfolgt die Umsetzung mit einem Monoisocyanat. Beispiele für solche Mono-isocyanate sind z. B.: Alkylisocyanate wie Benzylisocyanat, Methylisocyanat, Äthylisocyanat, n-Butylisocyanat, Isoamylisocyanat, n-Amylisoeyanat, Hexylisocyanat, n-Octylisocyanat, Isooctylisoeyanat, Dodecylisocyanat, Octadecylisocyanat, oder Arylisocyanate wie Phenylisocyanat, Tolylisocyanat, p-Äthylphenylisocyanat, -Cetylphenylisocyanat p-Dodecylphenylisocyanat 4-Dodecyl-2-Methylphenylisocyanat Xylylisocyanat, Naphthylisocyanat, p-chlorphenylisocyanat, m-Ghlorphenylisocyanat, oder Cycloalkylisocyanate wie Cyclohexylisocyanat, sowie 3-(Triethoxysilyl)propyl isocyanate (ICPTES). Auch diese Aufzählung erhebt keinen Anspruch auf Vollständigkeit.Besonders bevorzugt ist 3-(Triethoxysilyl) propyl isocyanate (ICPTES) und n-Butylisocyanat.

Dabei sind Einwaagenverhältnisse von Dimethacrylat mit einem aromatischen Kern und mindestens zwei Hydroxy-Gruppen zu Monoisocyanat von 1 mol: 1.01 mol bis 1 mol: 1.99 mol möglich. Bevorzugt sind Einwaagenverhältnisse von Dimethacrylat mit einem aromatischen Kern und mindestens zwei Hydroxy-Gruppen zu Monoisocyanat von 1 mol: 1.05 mol bis 1 mol:1.6 mol , besonders bevorzugt 1 mol: 1.2 mol bis 1 mol:1.5 mol.

Da dieses Reaktionsprodukt über eine sehr hohe Viskosität verfügt, ist es erforderlich, die Viskosität durch die Zugabe von niederviskosen Monomeren zu erniedrigen. Als radikalisch polymerisierbare Monomere eignen sich insbesondere mono- und polyfunktionelle (Meth)acrylate, d.h. Verbindungen mit einer oder mehreren (Meth)acrylatgruppen. Die nachfolgend genannten Verbindungen sind besonders bevorzugt: ethoxyliertes Bisphenol-A-Dimethacrylat, Urethandimethacrylate, Mono-, Di-, Tri- und Tetraethylenglycoldimethacrylate, Neopentylglycoldimethacrylate, 1,3-Butandioldimethacrylat, 1,6-Hexandioldimeth-acrylat, 2,2-Bis[4-(2-hydroxy-3-acryloyloxypropan)phenyl]-propan oder 2,2-Bis[4-(acryloyloxy-ethoxy)phenyl]propan. Neben den vorstehend genannten Dimethacrylaten sind auch die entsprechenden Diacrylate bevorzugt. Von den obigen Monomeren sind diejenigen mit zwei oder mehr (Meth)acrylatgruppen besonders bevorzugt, ebenso wie Monomermischungen, die mindestens ein Monomer mit zwei oder mehr (Meth)acrylatgruppen als Vernetzer enthalten. Ebenfalls geeignet sind Acrylamide oder Hydroxyalkylacrylamide. Radikalisch poymerisierbare Monomere mit einer Säurefunktion eignen sich ebenfalls für einen Einsatz. Radikalisch poymerisierbare Monomere mit einer Säurefunktion umfassen Oligomere und Polymere mit ethylenischer Ungesättigtheit und Säure- und / oder Säurevorstufenfunktionalität. Mit Säurevorstufen sind z.B. Anhydride, Säurehalogenide und Pyrophosphate gemeint.

Die erfindungsgemäßen dentalen Restaurationsmaterialien enthalten vorzugsweise auch einen oder mehrere Füllstoffe. Als Füllstoffe können praktisch alle bekannten und bewährten Füllstoffe eingesetzt werden, die bisher in Dentalmaterialien Verwendung fanden, wobei die Größe der Füllstoffpartikel bevorzugt unter 200 µm, vorzugsweise im Bereich von 10 nm bis 200 µm liegen und der Füllstoff vor der Einarbeitung in die Dentalmaterialien silanisiert werden sollte, bevorzugt mit γ-Methacryloxypropyltrimethoxysilan.

Zu den bevorzugten Füllstoffen zählen insbesondere anorganische Glas-, Glaskeramik- und Keramikpulver mit einer durchschnittlichen Partikelgröße von 0,01 bis 10 µm, bevorzugt 0,1 bis 5 µm, wie z.B. feinteilige Borsilikatgläser, Yb-, Zr- und La- Gläser, Alkali- und Erdalkalisilikate, sowie röntgenopake Füllstoffe, wie z.B. Ytterbiumtrifluorid und Wismutcarbonat, pyrogene Kieselsäure und Fällungskieselsäure, gefüllte und angefüllte organische Füllstoffe auf der Basis gemahlener Poly(meth)acrylate, Polycarbonate oder Polyepoxide sowie Mischungen der genannten Materialien. Füller auf der Basis gehärteter organischer Materialien werden auch als Isofüller bezeichnet. Die Partikelgröße von Isofüllern liegt vorzugsweise im Bereich von > 5 µm bis 200 µm, insbesondere > 5 µm bis 50 µm. Eine weitere Gruppe bevorzugter Füllstoffe stellen die Zeolithe dar, wobei Zeolithe vom Typ A besonders bevorzugt sind.

Die erfindungsgemäßen dentalen Restaurationsmaterialien enthalten vorzugsweise einen Initiator für die radikalische Polymerisation. Als Initiatoren für die radikalische Polymerisation eignen sich besonders Dibenzoylperoxid (DBPO), Di-p-Chlorbenzoylperoxid, tert.-Butylperoxybenzoat und Cumolhydroperoxid. Ebenso bevorzugt sind Initiatoren für die Photopolymerisation, wie Campherchinon. Weitere geeignete Photoinitiatoren werden in der US 4,746,686 beschrieben. Bevorzugte Initiatoren sind Dibenzoylperoxid und Campherchinon.

Die Initiatoren, wie zum Beispiel die genannten Peroxide, werden vorzugsweise in Kombination mit Polymerisationsakzeleratoren eingesetzt. Bevorzugte Beschleuniger sind tertiäre Amine, wie Triethanolamin, N,N,3,5-Tetramethylanilin, Dimethylamino-benzoesäureester, Dimethyl-p-toluidin oder Dihydroxyethyl-p-toluidin.

Werden die Peroxide allein eingesetzt, werden heißhärtende, in Kombination mit Beschleunigern kalthärtende oder selbsthärtende Materialien erhalten. Durch die Kombination von Photoinitiatoren mit Initiatoren für die Heiß- oder Kalthärtung werden dual härtbare Werkstoffe zugänglich.

Neben den genannten Bestandteilen können die erfindungsgemäßen dentalen Restaurationsmaterialien Stabilisatoren und Additive enthalten, wie z.B. Mittel zur Einstellung der Viskosität, Pigmente, Weichmacher und antimikrobielle Zusatzstoffe.

Als Stabilisatoren sind Benzochinon, Hydrochinon, Hydrochinonmonomethylether, 2,6-Di-tert.-butyl-p-kresol (butyliertes Hydroxytoluol, BHT), Phenol und phenolische Verbindungen sowie Chloranil bevorzugt. Die Stabilisatoren sollen eine vorzeitige Polymerisation des Werkstoffs verhindern. Bevorzugter Stabilisator ist 2,6-Di-tert.-butyl-p-kresol.

Darüber hinaus kann das Reaktionsprodukt aus einem mit einem aromatischen Kern und mindestens zwei Hydroxy-Gruppen bestehenden Dimethacrylat mit 2 unterschiedlichen Isocyanaten auch in dentalen Adhäsiven eingesetzt werden. Hier können zusätzlich zu den oben genannten Beispielen auch noch Lösungsmittel zum Einsatz kommen. Geeignete Lösungsmittel wären Aceton, Waser, Ethanol, Isopropanol oder Mischungen aus diesen Lösungsmitteln.

Die erfindungsgemäßen dentalen Restaurationsmaterialien eignen sich besonders zur Herstellung von Dentalmaterialien, wie Füllungskunststoffe, Zemente, beispielsweise von selbsthaftenden Befestigungszementen, und insbesondere von Adhäsiven.

Die Erfindung bezieht sich nicht nur auf den Dentalwerkstoff, sondern auch auf daraus hergestellte Fertigteile, z.B. künstliche Zähne, Veneers, Inlays, Onlays etc.. Darüber hinaus kann es auch in Zusammensetzungen eingesetzt werden, welche im 3D-Druckverfahren eingesetzt werden.

Nachfolgend wird die Erfindung anhand von einem Beispiel erläutert. Abbildung 1 zeigt das Herstellungsschema des erfindungsgemäßen Reaktionsprodukt aus einem mit einem aromatischen Kern und mindestens zwei Hydroxy-Gruppen bestehenden Dimethacrylat mit 2 unterschiedlichen Isocyanaten. Der Einfachheit halber wurden hier stellvertetend für die anderen möglichen Beispiele BisGMA, HDI und n-Butylisocyanat als Reaktionsteilnehmer ausgewählt. 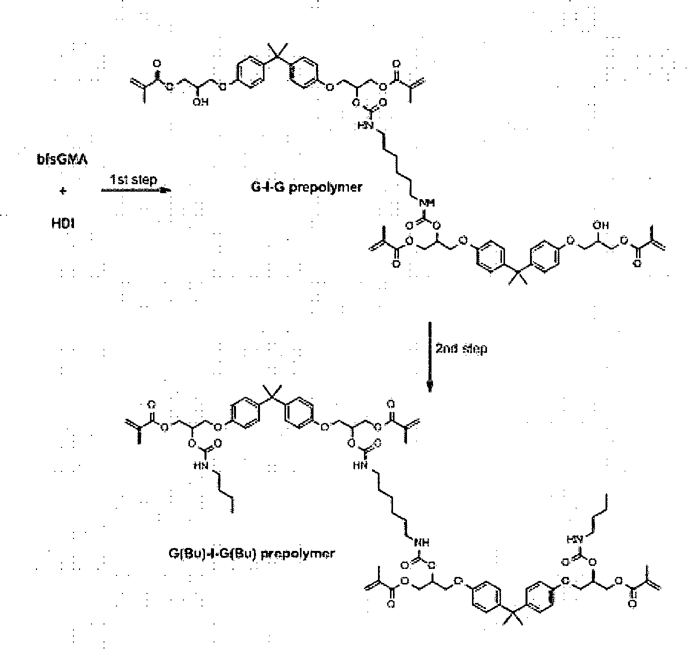 **Abbildung 1** : Herstellungsschema des erfindungsgemäßen Reaktionsprodukt aus einem mit einem aromatischen Kern und mindestens zwei Hydroxy-Gruppen bestehenden Dimethacrylat mit 2 unterschiedlichen Isocyanaten

### Herstellungsbeispiel 1:

Zur Verringerung der Viskosität des Reaktionsmediums wurde eine Mischung aus Bis-GMA- und Bis-EMA (Verhältnis 0.3 / 0.7 Gew./Gew.) eingesetzt. Sie wurde mit einer Menge an Stabilisator (BHT (0.1% w/w) und Katalysator (Dibutylzinn dilaurat 0.02% mol) versehen und bis auf 55-60 °C erhitzt. Die Mischung wurde dann in einen Reaktionskolben gegeben und unter Schutzgas (N₂ 96%/ O₂ 4% Atmosphäre) gesetzt. Danach wurde das System auf 80°C erhitzt und das Dilsocyanat-Reagenz (HDI; Verhältnis 1/0.4 mol/mol (Bis-GMA/HDI mol/mol) hinzugefügt. Die Mischung wurde 3 Stunden bei 80°C gerührt. Danach wurde in einem zweiten Schritt n-Butylisocyanat (Bis-GMA/n-Butylisocyanat Verhältnis 1 : 1.2 (mol/mol) hinzugefügt und ebenfalls 3 Stunden gerührt. Das erhaltene mittelviskose Produkt zeigte eine Viskosität von etwa 35 Pa*s.

25 g dieses Reaktionsproduktes wurden mit 62.5 g einer silanisierten Glaskeramik 8235 (Bariumaluminiumglas [Schott], mittlere Partikelgrösse 0.7 µm), 0.5 g Aerosil 200 [Evonik], 5 g Aerosil R8200 [Evonik], 0.075 g Campherchinon, 0.121 g Genocure EPD [Rahn] zu einer Masse gemischt und anschließend unter Vakuum verknetet. Von dem Material wurden Prüfkörper mit den Abmessungen 2x2x25 mm präpariert, die durch zweimaliges Bestrahlen für je 3 Minuten mit einer dentalen Lichtquelle (Hilite Power, Heraeus Kulzer) gehärtet wurden. Von den gehärteten Prüfkörpern wurde anschließend die mechanischen Eigenschaften nach der ISO Norm 4049 ermittelt. Für die Biegefestigkeit wurde dabei ein Mittel-Wert von 162 MPa erreicht.

### Herstellungsbeispiel 2:

Eine Mischung aus Bis-GMA- und Bis-EMA (Verhältnis 0.35 / 0.65 Gew./Gew.) wurde als Reaktionsmedium eingesetzt. Sie wurde mit einer Menge an Stabilisator (BHT (0.1% w/w) und Katalysator (Dibutylzinn dilaurat 0.02% mol) versehen und bis auf 55-60 °C erhitzt. Die Mischung wurde dann in einen Reaktionskolben gegeben und unter Schutzgas (N₂ 96% / O₂ 4% Atmosphäre) gesetzt. Danach wurde das System auf 80 °C erhitzt und das Dilsocyanat-Reagenz (TMDI; Verhältnis 1/0.3 mol/mol (Bis-GMA/TMDI mol/mol) hinzugefügt. Die Mischung wurde 3 Stunden bei 80 °C gerührt. Danach wurde in einem zweiten Schritt 3-(Triethoxysilyl)propyl isocyanate (ICPTES, Bis-GMA/ICPTES Verhältnis 1:1.4 (mol/mol)) hinzugefügt und ebenfalls 3 Stunden gerührt. Das erhaltene mittelviskose Produkt zeigte eine Viskosität von etwa 17 Pa*s.

25 g dieses Reaktionsproduktes wurden mit 62.5 g einer silanisierten Glaskeramik 8235 (Bariumaluminiumglas [Schott] mittlere Partikelgrösse 0,7 µm), 0.5 g Aerosil 200 [Evonik], 5 g Aerosil R8200 [Evonik], 0.075 g Campherchinon, 0.121 g Genocure EPD [Rahn] zu einer Masse gemischt und anschließend unter Vakuum verknetet. Von dem Material wurden Prüfkörper mit den Abmessungen 2x2x25 mm präpariert, die durch zweimaliges Bestrahlen für je 3 Minuten mit einer dentalen Lichtquelle (Hilite Power, Heraeus Kulzer) gehärtet wurden.Von den gehärteten Prüfkörpern wurde anschließend die mechanischen Eigenschaften nach der ISO Norm 4049 ermittelt. Für die Biegefestigkeit wurde dabei ein Mittel-Wert von 134 MPa erreicht.

### Herstellungsbeispiel 3:

Eine Mischung aus Bis-GMA- und Bis-EMA (Verhältnis 0.5 / 0.5 Gew./Gew.) als Reaktionsmedium wurde eingesetzt. Sie wurde mit einer Menge an Stabilisator (BHT (0.1% w/w) und Katalysator (Dibutylzinn dilaurat 0.02% mol) versehen und bis auf 55-60 °C erhitzt. Die Mischung wurde dann in einen Reaktionskolben gegeben und unter Schutzgas (N₂ 96% / O₂ 4% Atmosphäre) gesetzt. Danach wurde das System auf 80°C erhitzt und das Dilsocyanat-Reagenz (HDI; Verhältnis 1/0.3 mol/mol (Bis-GMA/HDI mol/mol) hinzugefügt. Die Mischung wurde 3 Stunden bei 80°C gerührt. Danach wurde in einem zweiten Schritt 3-(Triethoxysilyl)propyl isocyanate (ICPTES, Bis-GMA/ICPTES Verhältnis 1:1.3 (mol/mol)) hinzugefügt und ebenfalls 3 Stunden gerührt. Das erhaltene mittelviskose Produkt zeigte eine Viskosität von etwa 63 Pa*s.

25 g dieses Reaktionsproduktes wurden mit 62.5 g einer silanisierten Glaskeramik 8235 (Bariumaluminiumglas [Schott] mittlere Partikelgrösse 0,7 µm), 0.5 g Aerosil 200 [Evonik], 5 g Aerosil R8200 [Evonik], 0,075 g Campherchinon, 0,121 g Genocure EPD [Rahn] zu einer Masse gemischt und anschließend unter Vakuum verknetet. Von dem Material wurden Prüfkörper mit den Abmessungen 2x2x25 mm präpariert, die durch zweimaliges Bestrahlen für je 3 Minuten mit einer dentalen Lichtquelle (Hilite Power, Heraeus Kulzer) gehärtet wurden.Von den gehärteten Prüfkörpern wurde anschließend die mechanischen Eigenschaften nach der ISO Norm 4049 ermittelt. Für die Biegefestigkeit wurde dabei ein Mittel-Wert von 110 MPa erreicht.

Um Aussagen über die Degradationsprodukte zu erhalten wurden polymerisierte Materialien in Gegenwart und Abwesenheit des Enzyms Cholesterin-esterase inkubiert. Die Zusammensetzung der Degradationsprodukte von dem gehärteten Materialien wurde mittels Umkehrphasen-Flüssigkeitschromatographie gekoppelt mit einem Flugzeitmassenspektrometer bestimmt und die austretenden Verbindungen mittels Kernspinresonanz quantifiziert. Hintergrundinformationen finden sich in den folgenden Publikationen: Fujisawa S, Nuclear magnetic resonance spectra of Bis-GMA and Iso-bis-GMA. Dent Mater J. 1994 Dec;13(2):251-5; Vankerckhoven H, Lambrechts P, van Beylen M, Vanherle G., Characterization of composite resins by NMR and TEM., J Dent Res. 1981 Dec;60(12):1957-65. ; Asmussen E., NMR-analysis of monomers in restorative resins. Acta Odontol Scand. 1975;33(3): 129-34.

Die Versuchsanordnung wird im folgenden näher beschrieben:

### Enzym Vorbereitung:

Cholesterinesterase aus Pseudomonas sp. wurden in den gewünschten Konzentrationen in phosphatgepufferter Kochsalzlösung rekonstituiert. Die hergestellten Enzymlösungen, die zur Auffrischung der Enzymaktivität in den biologischen Abbauexperimenten verwendet wurden, wurden bei -20°C bis zum Gebrauch gelagert.

### Probenherstellung:

Es wurden zylindrische, scheibenförmige Probekörper mit einem Durchmesser von 23 mm und einer Höhe von 1 mm hergestellt.. Die Proben wurden mit einer Uni-XS-Lichthärtungseinheit (Heraeus-Kulzer) für 2 min gehärtet.

### Biostabilitätstest

Nach der Polymerisation wurden die gehärteten Pellets (Oberflächenbereich ∼ 850 mm²) in 2 ml D-PBS (Dulbecco's phosphatgepufferte Kochsalzlösung) bei 37 ° C unter Rühren für 24 Stunden inkubiert, um nicht umgesetzte Monomere zu entfernen. Die Pellets wurden dann mit destilliertem Wasser gespült und in Gegenwart von Cholesterinesterase für einen Zeitraum von 5 Tagen inkubiert. Kontrolllösungen von künstlichem Speichel und Ethanol wurden bei 37°C für die gleiche Zeitperiode gelagert und verwendet, um einen spektralen Leerwert zu liefern.

Ein Teil der Probe (1 ml) wurde vor HPLC-Messungen filtriert, um die Matrix zu entfernen, und der zweite Teil (1 ml) wurde lyophilisiert (Speedvac von THERMO scientific) für quantitative NMR-Messungen.

### NMR-Messungen

Etwa 10 mg lyophilisiertes Abbauprodukt wurden in deuteriertem Wasser gelöst. Genau 0,261 mg Dimethylsulfon wurden als interner Standard zur Quantifizierung zugegeben. Nach der Protonenanalyse wurden alle Integrale als mg Wasserstoffe zusammengefasst und quantifiziert. Ein mittleres Verhältnis (7,5%) von Wasserstoffatomen zu Kohlenstoff und Sauerstoff des erwarteten Abbauprodukts wurde gewählt und die resultierende Masse wurde berechnet. Mit dieser Technik konnte eine Sicherheit von ca. 90% erreicht werden und hatte den Vorteil, dass die Strukturen der ausgelaugten Verbindungen nicht bekannt sein mussten und keine Standards erforderlich sind.

Kommerzielle Dentalmaterialien (Spectrum TPH und Ceram.X universal [beide: Hersteller Firma Dentsply Sirona, York], Synergy D6 [Coltene-Whaledent AG, Altstätten], Admira Fusion [Voco,GmbH, Cuxhaven], Filtek Supreme XTE [3M Espe, Seefeld], Tetric evoceram [Ivoclar, Schaan]) und neu entwickelte Harz-Komposites wurden getestet. Die austretenden Verbindungen (Degradationsprodukte) aus den untersuchten Materialien erreichten signifikant unterschiedliche Mengen. In Tabelle 1 sind die Ergebnisse dargestellt.

Spectrum TPH und Ceram.X universal enthalten das oben erwähnte Urethan-modifiziertes BisGMA (ubis-system). Admira Fusion ist ein ormocer-basiertes dentales Füllungsmaterial. Die anderen Produkte repräsentieren am Markt etablierten Materialien. Deutlich ist zu erkennen, dass die neu entwickelten Komposite signifikant weniger Degradationsprodukte gegenüber dem Stand der Technik liefern.

**Tabelle 1 Quantitative Erfassung der Degradationsprodukte von ausgewählten dentalen Füllungskunststoffen und neu-entwickelten Füllungskompositen (Inkubationszeit 5 Tage). Angegeben ist die Menge Abbauprodukt in µg pro 2 g Komposit-Ausgangsmaterial.**

| Material | Chargennummer | Gesamtmasse Degradationsprodukte durch NMR [µg] |
|---|---|---|
| Neu entwickeltes Komposite (obiges Beispiel 1) | KTI 21 | 46 |
| Neu entwickeltes Komposite (obiges Beispiel 2) | KTI 9 | 30 |
| Neu entwickeltes Komposite (obiges Beispiel 3) | KTI 27 | 20 |
| Spectrum TPH [Dentsply] | 1601000829 | 89 |
| Synergy D6 [Coltene] | H27372 | 228 |
| Admira Fusion [Voco] | 1601556 | 81 |
| Ceram.X universal [Dentsply] | 1606000568 | 98 |
| Filtek Supreme XTE [3M Espe] | N740133 | 199 |
| Tetric evoceram [Ivoclar Vivadent] | V16175 | 216 |

## Patentansprüche

1. Reaktionsprodukt aus einem mit einem aromatischen Kern und mindestens zwei Hydroxy-Gruppen bestehenden Dimethacrylat mit 2 unterschiedlichen Isocyanaten.

2. Reaktionsprodukt aus einem mit einem aromatischen Kern und mindestens zwei Hydroxy-Gruppen bestehenden Dimethacrylat mit 2 unterschiedlichen Isocyanaten, wobei ein Isocyanat ein Diisocyanat ist.

3. Reaktionsprodukt nach Anspruch 2, wobei die Einwaagenverhältnisse von Dimethacrylat mit einem aromatischen Kern und mindestens zwei Hydroxy-Gruppen zu Diisocyanat von 1 mol: 0.01 mol bis 1 mol: 0,9 mol sind.

4. Reaktionsprodukt nach Anspruch 2, wobei die Einwaagenverhältnisse von Dimethacrylat mit einem aromatischen Kern und mindestens zwei Hydroxy-Gruppen zu Diisocyanat von 1 mol: 0.2 mol bis 1 mol :0,5 mol sind.

5. Reaktionsprodukt nach Anspruch 2, wobei die Einwaagenverhältnisse von Dimethacrylat mit einem aromatischen Kern und mindestens zwei Hydroxy-Gruppen zu Diisocyanat von 1 mol: 0.3 mol bis 1 mol :0,4 mol sind.

6. Reaktionsprodukt aus einem mit einem aromatischen Kern und mindestens zwei Hydroxy-Gruppen bestehenden Dimethacrylat mit 2 unterschiedlichen Isocyanaten, wobei ein Isocyanat ein Diisocyanat und das andere ein Monoisocyanat ist.

7. Reaktionsprodukt nach Anspruch 6, wobei die Einwaagenverhältnisse von Dimethacrylat mit einem aromatischen Kern und mindestens zwei Hydroxy-Gruppen zu Monoisocyanat von 1 mol : 1.01 mol bis 1 mol : 1.99 mol sind.

8. Reaktionsprodukt nach Anspruch 6, wobei die Einwaagenverhältnisse von Dimethacrylat mit einem aromatischen Kern und mindestens zwei Hydroxy-Gruppen zu Monoisocyanat von 1 mol: 1.05 mol bis 1 mol:1.6 mol sind.

9. Reaktionsprodukt nach Anspruch 6, wobei die Einwaagenverhältnisse von Dimethacrylat mit einem aromatischen Kern und mindestens zwei Hydroxy-Gruppen zu Monoisocyanat von 1 mol: 1.2 mol bis 1 mol :1.5 mol sind.

10. Reaktionsprodukt aus einem mit einem aromatischen Kern und mindestens zwei Hydroxy-Gruppen bestehenden Dimethacrylat mit 2 unterschiedlichen Isocyanaten, wobei das aus einem aromatischen Kern und mindestens zwei Hydroxy-Gruppen bestehende Dimethacrylat BISPHENOL A GLYCEROLATE DIMETHACRYLATE (BisGMA) ist.

11. Zusammensetzung, **dadurch gekennzeichnet, daß** sie ein Reaktionsprodukt nach einem der Ansprüche 1-10 enthält.

12. Zusammensetzung nach Anspruch 11, dadurch gekenzeichnet, daß sie zusätzlich ein radikalisch polymerisierbares Monomer enthält.

13. Zusammensetzung nach Anspruch 11, **dadurch gekennzeichnet, daß** sie als radikalisch polymerisierbares Monomer ein Methacrylat enthält.

14. Zusammensetzung nach Anspruch 11, **dadurch gekennzeichnet, daß** sie als radikalisch polymerisierbares Monomer ein Dimethacrylat enthält.

15. Zusammensetzung nach Anspruch 11, **dadurch gekennzeichnet, daß** sie als radikalisch polymerisierbares Monomer ein Ethoxyliertes Bisphenol A-Dimethaciylat enthält.

16. Zusammensetzung nach einem der Ansprüche 11 bis 15, **dadurch gekennzeichnet, daß** sie zusätzlich einen Initiator für die radikalische Polymerisation enthält.

17. Zusammensetzung nach einem der Ansprüche 11 bis 16, **dadurch gekennzeichnet, daß** sie zusätzlich Füllstoff enthält.

18. Zusammensetzung nach einem der Ansprüche 11 bis 17, **dadurch gekennzeichnet, daß** sie zusätzlich Lösungsmittel enthält.

19. Zusammensetzung nach einem der Ansprüche 11 bis 18, **dadurch gekennzeichnet, daß** sie
a) 0,5 bis 70 Gew.-% Reaktionsprodukt nach Anspruch 1 oder 2;
b) 0,01 bis 15 Gew.-% Initiator für die radikalische Polymerisation;
c) 0 bis 80 Gew.-% radikalisch polymerisierbares Monomer;
d) 0 bis 5 Gew. % Additive und Stabilisatoren
e) 0 bis 95 Gew.-% Lösungsmittel;
f) 0 bis 75 Gew.-% Füllstoff enthält.

20. Verwendung einer Zusammensetzung gemäß einem der Ansprüche 11 bis 19 als Material im Zusammenhang mit einem Medizinprodukt.

21. Verwendung einer Zusammensetzung gemäß einem der Ansprüche 11 bis 19 als Material für dentale Anwendungen.

22. Verwendung nach Anspruch 21 als dentales Füllungsmaterial.

23. Verwendung nach Anspruch 21 als dentales Adhäsiv.

24. Verwendung nach Anspruch 21 als dentaler Fissurenversiegler.

25. Verwendung nach Anspruch 21 als dentaler Befestigungszement.

26. Verwendung einer Zusammensetzung gemäß einem der Ansprüche 11 bis 19 als Harz für eine Digitaidruckanwendung.

27. Verwendung einer Zusammensetzung gemäß einem der Ansprüche 11 bis 19 als Grundlage für die Herstellung von Frässcheiben für die CAD/CAM-Anwendung.

28. Verwendung einer Zusammensetzung gemäß einem der Ansprüche 11 bis 19 als Grundlage zur Herstellung von in den menschlichen Körper implantierbaren Kunststoffen.
